# EUROPÄISCHE PATENTANMELDUNG

(11) **EP 1 752 193 A1**
(43) Veröffentlichungstag der Anmeldung: **14.02.2007**
(21) Anmeldenummer: 05017684.1
(22) Anmeldetag: 13.08.2005
(51) Int. Cl.: A61Q 19/00, A61Q 17/00, A61K 8/33, A01N 43/28, A01N 37/06, A01N 37/40, A01N 37/00, A01N 37/10, A01N 31/04, A01P 1/00

(54) **Verwendung eines oder mehrerer zyklischer 1,2-Alkylencarbonate zur Stabilisierung und Erhöhung der Wirksamkeit von Konservierungsstoffen und/oder-hilfen, sowie entsprechendes Mittel**

(71) Anmelder: Dr. Straetmans Chemische Produkte GmbH, 22143 Hamburg (DE)
(72) Erfinder: Straetmans, Udo, 22143 Hamburg (DE); Jänichen, Jan, 22143 Hamburg (DE); Petersen, Wilfried, 22143 Hamburg (DE); Kinder, Michael, 20253 Hamburg (DE)
(74) Vertreter: Fleck, Thomas

(57) **Zusammenfassung**

Die vorliegende Erfindung betrifft die Verwendung zyklischer 1,2-Alkylencarbonate zur Stabilisierung und Erhöhung der Wirksamkeit eines oder mehrerer das Wachstum von Mikroorganismen hemmenden Substanzen, sowie ein entsprechendes Mittel bzw. Mischung.
Erfindungsgemäße Stoffe, welche das Wachstum von Mikroorganismen hemmen können entweder Konservierungsmittel gemäß Anhang VI der EU-Kosmetikverordnung sein, aber auch multifunktionelle Additive mit antimikrobiellen Eigenschaften. Desweiteren bezieht sich die vorliegende Erfindung auf den Gebrauch dieser Mischungen als antimikrobiell wirksames System zur biologischen Stabilisierung von topischen Formulierungen, insbesondere in kosmetischen und dermatologischen Produkten.

## Beschreibung

### Hintergrund der Erfindung

Konservierungsmittel werden häufig gezielt in topischen Produkten, insbesondere in kosmetischen und dermatologischen Formulierungen, eingesetzt, in denen die Anwesenheit von Wasser und biologisch verwertbaren Materialien wie Fetten, Ölen und oberflächenaktiven Verbindungen eine ideale Matrix für das Wachstum von Bakterien, Hefen und Pilzen bilden.
Die Kontrolle des Wachstums von Mikroorganismen in diesen Produkten ist zwingend erforderlich, um sowohl die Wirksamkeit und Aussehen dieser Formulierungen zu erhalten, aber auch um die Sicherheit der Konsumenten zu gewährleisten.

In der Vergangenheit wurde der Konservierung von kosmetischen und dermatologischen Produkten wenig Aufmerksamkeit gewidmet. Die Kosten und die Effizienz eines Konservierungsmittels waren für den Hersteller der Produkte ausschlaggebend, und oft wurde mangelnde Produktionshygiene durch den exzessiven Gebrauch von hochwirksamen Konservierungsmitteln kompensiert. Zusätzlich waren die Kriterien zur Bestimmung der mikrobiologischen Stabilität so anspruchsvoll, dass exzessive Gebrauchsmengen von hochaktiven und billigen Chemikalien wie Formaldehyd/Formaldehyd-Abspaltern und / oder Isothiazolinonen ihren Weg in kosmetische und dermatologische Mischungen und in den Annex VI der kosmetischen Direktive fanden. Als Konsequenz aus dem exzessiven Gebrauch dieser chemisch wirkenden Konservierungsmittel wurde über eine steigende Anzahl von Reizungen und Sensibilisierungen berichtet. Konsequenterweise sind viele der traditionellen Konservierungsmittel daher für die Verwendung in kosmetischen Formulierungen eingeschränkt, und ihr öffentliches Ansehen hat stark gelitten.

In Folge einer allgemein steigenden Sensibilität gegenüber Chemikalien in Produkten des täglichen Bedarfs sind auch Konservierungsmittel für kosmetische und dermatologische Produkte ins Bewusstsein der Öffentlichkeit gelangt. Die Forderung der Kunden nach zunehmend natürlicheren Produkten zwang daher die Hersteller, sich auch nach Alternativen zu den traditionellen Konservierungsmitteln umzusehen, die gleichzeitig neue und zusätzliche Wirkungen bieten. "Soviel wie nötig und so wenig wie möglich" wurde zum Motto vieler Kosmetikproduzenten, und die Konservierung wird heutzutage in die gesamte Planung und Konzeptionierung eines kosmetischen oder dermatologischen Produktes implementiert. Zusätzlich ist der Bedarf an konservierungsmittelfreien Produkten gestiegen, was wiederum eine große Herausforderung an die Produzenten von kosmetischen und dermatologischen Produkten darstellt.

Nichtsdestotrotz darf aus Gründen der Produkt- und Konsumentensicherheit nicht völlig auf Maßnahmen zur biologischen Stabilisierung eines kosmetischen oder dermatologischen Produktes verzichtet werden. In vielen Produkten kann zur Kontrolle des Wachstums von Mikroorganismen auf die herkömmlichen Konservierungsmittel nicht verzichtet werden, und das Risiko für den Konsumenten ist bei einer vernünftigen Konzentration der Konservierungsmittel im Produkt geringer als ein ungenügender Schutz des Produktes. Als Ergebnis einer Abschätzung, welchem Risiko die Konsumenten ausgesetzt sind, ist es daher erstrebenswert, biologisch stabile kosmetische und dermatologische Produkte mit reduziertem Gehalt an Konservierungsmitteln herzustellen, um die unerwünschten Nebeneffekte der Konservierungsmittel zu reduzieren.

Überraschenderweise wurde nun gefunden, dass durch den Zusatz von zyklischen 1,2-Alkylencarbonaten, insbesondere 1,2-Alkylencarbonaten mit einer Kettenlänge von n = 2 bis 8, vorteilhafterweise 4 - 8, zu kosmetischen und dermatologischen Produkten die Effizienz von Konservierungsmitteln erheblich gesteigert werden kann und so der Forderung nach einer Reduktion von Konservierungsmitteln in diesen Produkten ohne Vernachlässigung der Produktsicherheit nachgekommen werden kann.

Die genannten 1,2-Alkylencarbonate haben sich als sichere Rohstoffe für kosmetische und dermatologische Produkte herausgestellt, da sie toxikologisch unbedenklich sind, eine gute Hautverträglichkeit aufweisen, in den üblichen kosmetischen und dermatologischen Formulierungen chemisch stabil und von unauffälligem Geruch sind sowie preiswert durch Standardverfahren aus einfachen Vorstufen herstellbar sind. Untersuchungen zu den hauttoxikologischen Eigenschaften der zyklischen Alkylencarbonate haben ein hohes Maß an Hautfreundlichkeit sogar an sensiblen Hautpartien wie z.B. den Augen deutlich gemacht. Dies macht diese Verbindungen zu idealen Bestandteilen von topischen Anwendungen, insbesondere von dermatologischen und kosmetischen Produkten.

### Stand der Technik

Zyklische 1,2-Alkylencarbonate können leicht aus den ensprechenden 1,2-Alkandiolen durch deren Umsetzung mit Dimethyl- oder Diethylcarbonate in Gegenwart von Basen wie z.B. Kaliumcarbonat hergestellt werden. 1,2-Butylencarbonat wird unter dem Handelsnamen Jeffsol^{®} BC von der Fa. Huntsman Inc. angeboten.
Zyklische 1,2-Alkylencarbonate wurden bisher in verschiedenen technischen Anwendungen, insbesondere als nicht-wässrige Lösungsmittel für elektrochemische Verfahren und als Lösungsmittel für Farbentferner (z.B. US 2002/111284, US 2000/654438), beschrieben. Insbesondere Glycerylcarbonat ist als Lösungsmittel geeignet (DE 10110855).
In landwirtschaftlichen Anwendungen wurden Propylencarbonat und Butylencarbonat als biologisch abbaubare, inerte und ungiftige Hilfsstoffe für Pestizidmischungen beschrieben.
Die Verwendung von Propylencarbonat in topischen Anwendungen in Nagellackentfernern (EP 0701811 und US 4,801,331), in Lippenstiften (US 5,843,407) und als ein Vehikel für dermatologisch wirksame Bestandteile in Salben (US 4,017,615) ist beschrieben. Die Verwendung in Feuchtreinigungstüchern wegen ihrer Hautfreundlichkeit von 1,2-Ethylencarbonat, 1,2-Propylencarbonat, 1,2-Butylencarbonat und 1,2-Glycerylcarbonat wurde beschrieben (WO 2002/087327). Glycerylcarbonat wird als Emulgator (DE 19950017, WO 9932216), als Synergist für Esterasehemmer in desodorierenden kosmetischen Mitteln (WO 2000-037035) und als Haut- und Haarkonditionierer (DE 19851451) beschrieben. Die feuchtigkeitsspendende Wirkung zyklischer Carbonate in kosmetischen Formulierungen wird in der Patentschrift EP 1112058 beansprucht.

### Gegenstand der vorliegenden Erfindung

Die vorliegende Erfindung betrifft Mischungen, die zyklische 1,2-Alkylencarbonate in Kombination mit einem oder mehreren das Wachstum von Mikroorganismen hemmenden Stoffe enthalten. Erfindungsgemäße Stoffe, die das Wachstum von Mikroorganismen hemmenden, können sowohl Konservierungsmittel gemäß Anhang VI der EU-Kosmetikverordnung sein aber auch multifunktionelle Additive mit antimikrobiellen Eigenschaften.
Ein weiterer Aspekt dieser Erfindung bezieht sich auf die Verwendung der betreffenden Mischungen zur biologischen Stabilisierung von Formulierungen zur Reinigung, Pflege und Behandlung oder Dekoration von Haut und Haaren. Ein weiterer Aspekt dieses Patents ist die Angabe von Mischungen, in denen der Anteil an Konservierungsmitteln zur Kontrolle des mikrobiellen Wachstums in den genannten Anwendungen allein nicht ausreichend wäre.

### Detaillierte Beschreibung der Erfindung

Die zyklischen 1,2-Alkylencarbonate, welche in diesem Patent beschrieben sind, können durch die folgende allgemeine chemische Strukturformel wiedergegeben werden: 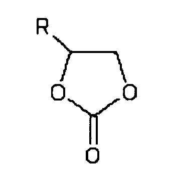 wobei R = -CH₂CH₃ oder lineare oder verzweigte Alkylketten mit 3 bis 6 Kohlenstoffatomen sein können. Es sei darauf hingewiesen, dass der Begriff zyklische 1,2-Alkylencarbonate im Rahmen des vorliegenden Textes sowohl die entsprechenden S- als auch die R-konfigurierten Enantiomere als auch die razemischen Gemische diesen S- und R-konfigurierten Enantiomeren umfasst. Aus kommerziellen Gründen ist es zwar besonders vorteilhaft, razemische Gemische der jeweiligen zyklischen 1,2-Alkylencarbonate einzusetzen, da diese synthetisch besonders leicht zugänglich sind, die reinen Enantiomere bzw. Diastereomere sind aber ebenfalls für die erfindungsgemäßen Zwecke geeignet.

Die Verwendung dieser zyklischen 1,2-Alkylencarbonate zur Herstellung von Mischungen mit herkömmlichen Konservierungsmitteln und der erfindungsgemäße Einsatz dieser Mischungen in kosmetischen oder dermatologischen Anwendungen zeigte überraschenderweise, dass die Effizienz der Konservierungsmittel in Konzentrationen, die für sich allein keine antimikrobielle Wirkung mehr zeigten, durch den Zusatz der zyklischen 1,2-Alkylencarbonate erheblich gesteigert werden kann und somit der Anteil der Konservierungsmittel in den entsprechenden Produkten signifikant gesenkt werden konnte.

Zyklische 1,2-Alkylencarbonate, für die dieser synergistische Effekt nachgewiesen werden konnten, finden sich bevorzugt in der Gruppe der linearen und verzweigten 1,2-Alkylencarboate mit einer Kettenlänge von n = 4 - 8, z.B. 1,2-Butylencarbonat, 1,2-Pentylencarbonat, 1,2-Hexylencarbonat und 1,2-Octylencarbonat. Besonders ausgeprägt Effekte zeigen sich beim Butylencarbonat und dem Pentylencarbonat, weshalb diese Verbindungen besonders bevorzugt im Sinne der vorliegenden Erfindung sind.

Konservierungsmittel im Sinne der Erfindung, für die dieser synergistische Effekt nachgewiesen werden konnte, sind Methylparaben, Ethylparaben, Propylparaben, Isopropylparaben, Butylparaben und die Mischungen dieser Parabene, Phenoxyethanol, Benzoesäure und ihre Ester und Salze, Sorbinsäure und ihre Salze sowie Benzylalkohol.
Die zu erwartenden synergistischen Effekte von 1,2-Alkylencarbonaten mit Iodopropinylbutylcarbamat, Formaldehyd and Formaldehyd-Abspalter (z.B. DMDM-Hydantoin, Imidazolinylharnstoff, Paraformaldehyd), Chlorphenesin, Bronopol, Methyldibromglutaronitril, Dehydracetsäure und ihre Salze, Polyaminopropylbiguanide, Chlorhexidin und seine Derivate (z.B. Chlorhexidinacetat, Chlorhexidingluconat, Chlorhexidinhydrochlorid), Methylisothiazolinon und Methylchloroisothiazolinone neben den in Anhang 6, Teil A und B der Kosmetikverordnung aufgeführten weiteren Stoffklassen oder Mischungen der genannten Konservierungsmittel werden ebenfalls in dieser Erfindung beansprucht.

Multifunktionellen Kosmetikadditive mit antimikrobiellen Eigenschaften im Sinne dieser Erfindung finden sich vor allem in der Gruppe der mittelkettigen polaren Substanzen. Diese Gruppe umfasst Polyole mit 5 - 12 Kohlenstoffatomen, wie z.B. Pentylenglycol, Hexylenglycol, 1,2-Hexandiol, Caprylyl Glycol oder 2-Methyl-1,3-propandiol, Monoester aus Glycerin und Fettsäuren der Kettenlänge n = 6 - 12, wie z.B. Glyceryl Caprylate, Glyceryl Caprate oder Glyceryl Laurate, Ether der Glycerins mit linearen oder verzweigten Alkanolen der Kettenlänge n = 6 - 12, wie z.B. das Ethylhexylglycerin sowie N-Acylderivate von Aminosäuren, z.B. N.Undecenylglycin. Besonders ausgeprägte Effekte zeigten sich beim Caprylyl Glycol und beim Glyceryl Caprylate, weshalb diese Verbindungen besonders bevorzugt im Sinne der vorliegenden Erfindung sind.

vorzugsweise liegt die Einsatzkonzentration der erfindungsgemäßen Mischungen aus zyklischen 1,2-Alkylencarbonaten und Konservierungsmitteln bei Verwendung als Konservierungsmittelsystem für kosmetische oder dermatologische Anwendungen im Bereich zwischen 0,01 bis 50 Gewichts-%, besonders bevorzugt aber im Bereich zwischen 0,1 und 10 Gewichts-%, jeweils bezogen auf die Gesamtmasse des Endproduktes.
Die erfindungsgemäß verwendeten synergistischen Mischungen aus zyklischen 1,2-Alkylencarbonaten und Konservierungsmitteln lassen sich ohne Schwierigkeiten in gängige kosmetische oder dermatologische Formulierungen, wie z.B. Cremes, Salben, Lotionen, Tinkturen, Pumpsprays, Aerosolsprays, Nagellacke, Nagellackentferner, Nagelbalsame und dergleichen einarbeiten. Die synergistisch wirksame Mischungen enthaltenden kosmetischen oder dermatologischen/keratologischen Formulierungen können hierbei ansonsten wie üblich zusammengesetzt sein und zur Behandlung der Haut und / oder der Haare im Sinne einer dermatologisachen Behandlung im Sinne der pflegenden Kosmetik dienen. Die hier beschriebenen synergistischen Mischungen aus zyklischen Alkylencarbonaten und Konservierungsmitteln können aber auch in Schminkprodukten in der dekorativen Kosmetik eingesetzt werden.

Die kosmetischen und dermatologischen Formulierungen können kosmetische Hilfsstoffe enthalten sein, wie sie üblicherweise in solchen Formulierungen verwendet werden wie beispielsweise Feuchteregulierer und Benetzmittel, Tenside, Ölkörper, Emulgatoren, Perlglanzwachse, Konsistenzgeber, Verdickungsmittel, Überfettungsmittel, Stabilisatoren, Polymere, Siliconverbindungen Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektrepellentien, Selbstbräuner, Tyrosininhibitoren (Depigmentierungsmittel), Hydrotrope, Solubilisatoren, weitere Konservierungsmittel, Parfümöle, Farbstoffe etc., die nachstehend exemplarisch aufgelistet sind.

### Feuchteregulierer und Benetzmittel

Als Feuchteregulierer und Benetzmittel kommen hauptsächlich lineare oder verzweigte Polyole mit einer Kettenlänge von 4 bis 12 Kohlenstoffatomen in Frage, z.B. Pentylenglycol, 1,2-Hexandiol, Heptylenglycol, Caprylyl Glycol, Hexylenglycol, Methylpropandiol oder beliebige Mischungen der linearen oder verzweigten Polyole.

### Tenside

Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten. Der Anteil der Tenside liegt hier üblicherweise bei etwa 0,1 bis 30, vorzugsweise 5 bis 25 und insbesondere 10 bis 20 Gew.-%.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäurethionate, Fettsäuresarcoinate, Fettsäuretauride, N-Acylaminosäuren, wie beispielsweise Acyllactylate, Acyltartrate, Acylglutamate und Acylaspartate, Alkyloligoglucosidulfate, Proteinfettsäurekondensate (insbesondere planzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettsäurepolyglycerylester, Fettalkoholpolyglycolether, Alkylphenolpolyglycolether, Fettsäurepolyglycolester, Fettsäureamidpolyglycolether, Fettaminpolyglyolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternisierte Fettsäuretrialkanolaminestersalze. Typische Beispiel für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d. h. besonders hautverträgliche Tenside sind Fettalkoholpolyglycolethersulfate, Monoglyceridsulfate, Mono- und/oder Dialkylsulfosuccinate, Fettsäureisothionate, Fettsäuresarcosinate, Fettsäuretauride, Fettsäureglutamate, □-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Körperpflegemittel, wie Cremes, Lotionen und Milchen, enthalten üblicherweise eine Reihe von Ölkörpern und Emollients, die dazu beitragen die sensorischen Eigenschaften zu optimieren. Die Ölkörper sind üblicherweise in einer Gesamtmenge von 0,1 - 50 Gewichts-%, vorzugsweise 5 - 25 Gewichts-% und insbesondere 5 - 15 Gewichts-% enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, CVetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isosterylstearat, Isostearylisostearat, Isostearyloleat, Isostearylbehenat, Oleylmyristat, Oleylpalmitat, Oleylstearat, Oleylisostearat, Oleyloleat, Oleylbehenat, Oleylerucat, Behenylmyristat, Behenylpalmitat, Behenylpalmitat, Behenylstearat, Behenylisostearat, Behenyloleat, Behenylbehenat, Erucylmyristat, Erucylpalmitat, Erucylstearat, Erucylisostearat, Erucyloleat, Erucylbehenat und Erucylerucat in Betracht. Daneben eigenen sich auch Ester von linearen C₆-C₂₂-Fettsäuren mit unverzweigten oder verzweigten mehrwertigen Alkoholen wie z.B. Butylene Glycol Dicaprylat/Dicaprat (Dermofeel® BGC) oder Ester des 2-Methyl-1,3-propandiols, des 1,1,1-Trimethylolethans oder 1,1,1-Trimethylolpropans. Desweiteren eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis von C₆-C₁₂-Fettsäuren, wie z.B. Triheptanoin (Dermofeel® TC-7) oder Tricaprylin (Dermofeel® MCT), flüssige und feste Monoglyceride von Fettsäuren mit einer Kettenlänge von 6 bis 18 Kohlenstoffatomen wie z.B. Glycerylcaprylat (Dermosoft^{®} GMCY), Glycerylcaprat (Dermosoft^{®} GMC), oder Glyceryllaurat, flüssige und feste Diglyceride von Fettsäuren mit einer Kettenlänge von 6 bis 18 Kohlenstoffatomen, flüssige und feste Mono-/Di/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv^{®} TN), lineare oder verzweigte, symmetrische oder unsymmetrische Dialkylether mit 6 bis 22 Kohlenstoffatomen pro Alkylgruppe, wie z. B. Dicaprylyl Ether (Cetiol^{®} OE), Ringöffnungsprodukte von epoxidierten Fettsäureestern mit Polyolen, Siliconöle (Cyclomethicone, Sliciummethicontypen u. a.) und/oder aliphatische bzw. naphthenische Kohlenwasserstoffe, wie z. B. Squalan, Squalen oder Dialkylcyclohexane, lineare und / oder verzweigte Alkylether des Glyzerins (z.B. Ethylhexylglyzerin) oder Mischungen dieser Ölkörper in beliebigen Verhältnissen.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/oder 0 bis 5 Mol Propylenoxid an linear Fettalkohole mit 8 bis 22 C-Atomen, an Fettsäuren mit 1 bis 22 C-Atomen, an Alkylphenole mit 8 bis 15 C-Atomen in der Alkylgruppe sowie Alkylamine mit 8 bis 22 Kohlenstoffatomen im Alkylrest
- Alkyloligoglykoside mit 8 bis 22 Kohlenstoffatomen im Alkylrest und deren ethoxylierte Analoga
- Anlagerungsprodukte von 1 bis 15 Mol Ethylenoxid an Ricinusöl und/oder gehärtetes Ricinusöl
- Anlagerungsprodukte von 15 bis 60 Mol Ethylenoxid an Ricinusöl und/oder gehärtetes Ricinusöl
- Partialester von Glycerin und/oder Sorbitan mit ungesättigten, linearen oder gesättigten, verzweigten Fettsäuren mit 12 bis 22 Kohlenstoffatomenund/oder Hydroxyarbonsäuren mit 3 bis 18 Kohlenstoffatomen sowie deren Addukte mit 1 bis 30 Mol Ethylenoxid.
- Partialester von Polyglycerin (durchschnittlicher Eigenkondenstaionsgrad 2 bis 8), Polyethylenglycol (Molekulargewicht 400 bis 5000), Trimethylolpropan, Pentaerythrit, Zuckeralkoholen (z. B. Sorbit), Alkylglucosiden (z. B. Methylglucosid, Butylglucosid, Laurylglucosid) sowie Polyglucosiden (z. B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin
- Mono-, Di- und Trialkylphosphate sowie Mono-, Di- und/oder Tri-PEGalkylphosphate und deren Salze
- Wollwachsalkohole
- Polysiloxan-Polyalkyl-Polyether-Copolymere bzw. Entsprechende Derivate
- Block-Copolymere z. B. Polyethylenglycol-30-Dipolyhydroxystearat
- Polymeremulgatoren, z. B. Pemulen-Typen (TR-1, TR-2) von Goodrich
- Polyalkylenglycole sowie
- Glycerincarbonat.

### - Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und diester von Anlagerungsprodukten von Ethylenoxid an Glycerin sind als Rückfettungsmittel für kosmetische Zubereitungen bekannt.

### - Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansequioleat, Sorbitandioleat, Sorbitantrioleat, Sorbitanmonoerucat, Sorbitansesquierucat, Sorbitandierucat, Sorbitantrierucat, Sorbitanmonoricinolat, Sorbitansesquiricinolat, Sorbitandiricinolat, Sorbitantriricinolat, Sorbitanmonohydroxystearat, Sorbitanmonotartrat, Sorbitansesquitartrat, Sorbitanditartrat, Sorbitantritartrat, Sorbitanmonocitrat, Sorbitansesquitartrat, Sorbitandicitrat, Sorbitantricitrat, Sorbitanmonomaleat, Sorbitansesquimaleat, Sorbitandimaleat, Sorbitantrimaleat sowie deren technische Gemische . Ebenfalls geeignet sind Anlagerungsprodukte von 1 bis 30, vorzugsweise 5 bis 10 Mol Ethylenoxid an die genannten Sorbitanester.

### - Polyglycerinester

Typische Beispiele für geeignet Polyglycerinester sind Polyglyceryl-3 Stearate (Dermofeel^{®} PS), Polyglycerin-3-Palmitate (Dermofeel^{®} PP), Polyglyceryl-6 Caprylate (Dermofeel^{®} G 6 CY), Polyglyceryl-10 Laurate (Dermofeel^{®} G 10 L), Polyglyceryl-2-Laurate (Dermofeel^{®} G 2 L), Polyglyceryl-3-Laurate (Dermofeel^{®} G 3 L), Polyglyceryl-5-Laurate (Dermofeel^{®} G 5 L), Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} Gl 34), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Dermofeel^{®} PR) Polyglyceryl Dimerate Isostearate sowie deren Gemische. Beispiele für weitere geignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### - Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäure mit 12 bis 22 Kohlenstoffatomen, wie beispielsweise Azelainsäure oder Sebacinsäure.

### - Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- oder eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinate, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinate. Ebenfalls geeignet ist das unter der CTFA-Bezeichnung Cocoamidopropyl Betaine bekannte Fettsäureamid-Derivat. Weitere geeignete Emulgatoren sind ampholytische Tenside. Unter ampholyytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren N-Alkyliminidipropionäuren, N-Hydroxyethyl-N-alkylamidopropylglycin, N-Alkyltaurine, N-Alkylsarcosine, 2-Alkylaminopropionsäuren und Alkylminoessigsäuren mit jeweilsetwa 8 bis 18 C-Atomen in der Alkylgruppe. Beispiele für ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht.

### Fett und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d.h. feste oder flüssige, pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder - stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwchse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher häufig auch als Phosphatidylcholine (PC) bezeichnet. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierten Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 - 15 Kohlenstoffatomen und 2 bis 10 Hydroxylgruppen sowie deren Mischungen.

### Konsistenzgeber und Verdickungsmittel

Als weitere Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Bettracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (Hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und diester von Fettsäuren, Polyacrylate (z. B. Carbopole^{®} und Permulene-Typen von Goodrich; Synthalene^{®} von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z. B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit geeigneter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z. B. Magnesium-, Aluminium- und/oder Zinkstearat bzw. ricinoleat eingesetzt werden.

### Polymere

Geeignete kationische Polymere sind beispielsweise kationische Cellulosederivate, wie z. B. eine quaternierte Hydroxyethylcellulose, die unter der Bezeichnung Polymer JR 400® von Amerchol erhältlich ist, kationische Stärke wie sie z.B. von der Dr. Straetmans GmbH unter den Namen Amylomer angeboten wird, Copolymere von Diallylammoniumsalzen und Acrylamiden, quaternierte Vinylpyrrolidon/Vinilimidazol-Polymere, wie z. B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z. B. Amodimethicone, Copolymere der Adipinsäure und Dimethylaminohydroxypropyldiethylentriamin (Cartaretine®/Sandoz), Copolymere der Acrylsäure mit Dimethyldiallyl-ammoniumchlorid (Merquat® 550/Chemviron), Polyaminopolyamide sowie deren vernetzte wasserlösliche Polymere, kationische Chitinderivate wie beispielsweise quaterniertes Chitosan, gegebenenfalls mikrokristallin verteilt, Kondensationsprodukte aus Dihalogenalkylen, wie z. B. Dibrombutan mit Bisdialkylaminen, wie z. B. Bis-Dimethylamino-1,3-propan, ktionischer Guar-Gum, wie z. B. Jaguar® CBS, Jaguar® C-17, Jaguar® C-16 der Firma Celanese, quaternierte Ammoniumsalz-Polymere, wie z. B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.-Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, zyklische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter und Antioxidantien

Neben den neueren UV-Filtern, die sich durch gute Lichtschutzeigenschaften auszeichnen und die in der jüngsten Vergangenheit breiten Einsatz gefunden haben (z.B. der UV-B-Filter 2,4,6-Trianilino-p-(carbo-2'-ethylhexyl-1'oxy)-1,3,5-triazin (INCI: Ethylhexyl Triazon), der von der BASF unter dem Handelsnamen Uvinul T 150 vertrieben wird, der UV-A-Filter 1-(4-tert-butylphenyl)-3-(4-methoxyphenyl)-propan-1,3-dion (INCI: Butyl Methoxydibenzoylmethan), welcher z.B. unter dem Handelsnamen Parsol 1789 von der Fa. DSM angeboten wird oder das Bis-Octyloxyphenolmethoxyphenyltriazin (INCI: Bis-Ethylhexyloxyphenol Methoxyphenyl Triazine), ein Breitbandfilter, der z.B. unter dem Handelsnamen Tinosorb S von der Fa. Ciba Geigy (EP0775698 B1) angeboten wird) können weitere oder andere UV-Filter in den synergistisch wirksame Konservierungssysteme aus zyklischen Carbonaten und Konservierungsmittel enthaltenden kosmetischen oder dermatologischen Formulierungen vorliegen. UV-B-Filter können öllöslich oder wasserlöslich sein. Als öllösliche Substanzen sind z.B. zu nennen:
- 3-Benzylidencampher bzw. 3-3-Benzylidennorcampher und dessen Derivate, z.B. 3-(4-Methylbenzyliden)campher wie in der Patentschrift EP 0693471 B1 beschrieben.
- 4-Aminobenzoesäurederivate, vorzugsweise 4-(Dimethylamino)benzoesäure-2-ethylhexylester, 4-(Dimethylamino)benzoesäure-octylester und 4-(Dimethylamino)benzoesäure-2-amylester.
- Ester der Zimtsäure, vorzugsweise 4-Methoxyzimtsäure-2-ethylhexylester, 4-Methoxyzimtsäure-propylester, 4-Methoxyzimtsäureisoamylester oder 2-Cyano-3,3-phenylzimtsäure-2-ethylhexylester (Octocrylene)
- Ester der Salicylsäure, vorzugsweise Salicylsäure-2-ethylhexylester, Salicylsäure-4-isopropylbenzylester oder Salicylsäurehomomenthylester
- Derivate des Bezophenons, vorzugsweise 2-Hydroxy-4-methoxybezophenon, 2-Hydroxy-4'-methoxybezophenon, 2,2'-Dihydoxy-4-methoxybenzophenon
- Ester der Benzalmalonsäure, vorzugsweise 4-Methoxybenzmalonsäure-di-2-ethylhexylester
- Triazinderivate wie z.B. Dioctyl Butamido Triazon (Uvasorb HEB)
- Ketotricyclo(5.2.1.0)decan-Derivate, wie in der EP 0694521 B1 beschrieben.

Als wasserlösliche Substanzen kommen in Frage:
- 2-Phenylbenzimidazol-5-sulfonsäure und deren Alkali-, Erdalkali-, Ammonium-, Alkylammonium, Alkanolammonium- und Glucammoniumsalze
- Sulfonsäurederivate von Benzophenon, vorzugsweise 2-Hydroxy-4-methoxybenzophenon-5-sulfonsäure und ihre Salze
- Sulfonsäurederivate des Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-Phenyl-3-(4'isopropylphenyl)-propan-1,3-dion sowie Enaminverbindungen, wie beschrieben in der DE 19712033 A1. Die UV-A- und UV-B- Filter können selbstverständlich auch in Mischungen eingesetzt werden. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium, Alkanolammonium- und Glucammonumsalze kombiniert.

Neben den genannten löslichen Stoffen kommen für diesen Zweck auch unlösliche Lichtschutzpigmente, nämlich feindisperse Metalloxide bzw. salze in Frage. Beispiele für geeignete Metalloxide sind insbesondere Titandioxid und Zinkoxid und daneben Oxide des Eisens, Zirkoniums, Siliziums, Mangans, Aluminiums und Cers sowie deren Gemische. Als salze können Silicate (Talk), Bariumsulfat oder Zinkstearat eingesetzt werden. Die Oxide und Salze werden in Form der Pigmente für hautpflegende und hautschützende Emulsionen du dekorative Kosmetik verwendet. Die Partikel sollen dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobisiert vorliegen. Typische Beispiele sind gecoatete Titandioxide, wie z.B. Titandioxid T 805 der Fa. Degussa oder Eusolex T 2000 der Fa. Merck. Als hydrophobe Coatingmittel kommen dabei vor allem Silicone und dabei speziell Trialkoxyoctylsilane oder Simethicone in Frage. In Sonnenschutzmitteln werden bevorzugt so genannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn schädliche UV-Strahlung in die Haut eindringt. Typische Beispiele sind hierfür Aminosäure(z.B. Histidin, Tyrosin oder Tryptophan) und deren Derivate, Imidazole (z.B. Urocaninsäure) und deren Derivate, Peptide wie D,L-Carnosin, D-Carnosin, L-Carnosin und deren Derivate, Carotinoide, Carotine (z.B. α-Carotin, β-Carotin, Lycopin) und deren Derivate, Liponsäuren und deren Derivate (z.B. Dihydroliponsäure), Aurothioglucose, Propylthiouracil und andere Thiole (z.B. Thioredoxin, Glutathion, Cystein, Cystin, Cysteamin un deren Glycolsyl-, N-Acetyl-, Mehyl-, Ethyl-, Propyl-, Amyl-, Butyl-, Lauryl-, Palmitoyl-, Oleyl-, □-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximin, Homocysteinsulfoximin, Butioninsulfoine, Penta-, Hexa-, Heptathioninsulfoximin) in sehr geringen verträglichen Dosierungen (z.B. pmol bis µmol), ferner Komplexbildener wie z.B. α-Hydroxyfettsäuren, Palmitinsäure, Phytinsäure, Lactoferrin), α-Hydroxysäurem (z.B. Zitronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. □-Linolensäure, Linolsäure, Ölsäure), Folsäure und deren Derivate, Ubichinon und Ubichinol und deren Derivate, Vitamin C und Derivate (z.B. Ascorbyl Palmitat, Magnesium Ascorbylphosphat, Ascobylacetat), Tocopherole (z.B. α-Tocopherol, β-Tocopherol, γ-Tocopheol δ-Tocopherol) und deren Derivate (Tocopherly Acetat), Vitamin A und Derivate (Vitamin A Palmitat) soweie Koniferylbenzoat des Benzoeharzes, Rutinsäure und deren derivate, α-Glycosylrutin, Ferulasäure, Furfurylidengluticol, Carnosin, Butylhydrxytoluol, Butylhydroxyanisol, Nordihydroguarjakharzsäure, Nordihydroguarjaretsäure, Trihydroxybutyrophenon, Harnsäure und deren Derivate, Mannose und deren Derivate, Speroxid-Dismutase, Zink und dessen Derivate (ZnO, ZnSO₄), Selen un dessen Derivate (z.B. Selen-Methionin), Stilbene und deren Derivate (z.B. Stilbenoxid, trans-Stilbenoxid) und die errfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffn sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### - Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethylphenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### - Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder - phosphate, wie beispielsweise Lanosterin-, Cholesterin-, Campesterin-, Stigmasterin- und Sitosterinsulfat bzw. -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### - Geruchsabsorber

Als Geruchsabsorber eignen sich Stoffe, die geruchsbildende Verbindungen aufnehmen und weitgehend festhalten können. Sie senken den Partiladruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfumöle, die zusätzlich zu ihrer Funktion als Geruchsüberdecker den Deodorantien ihre jeweilige Duftnote verleihen. Als Parfumöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, p-tert.-Butylcyclohexylacetat, Linalylacetat, Phenylethylacetat, Linalylbenzoat, Benzylformiat, Allylcyclohexylpropionat, Styrallylpropinonat, und Benzylsalicylat. Zu den Ethern zähln beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jojone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten vrwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, □-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, □-Damascone, Geraniumöl Bourbon, Cyclohexylsalicylat, Vertofix Couer, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen eingesetzt.

### - Antitranspirantien

Antitranspirantien (Antiperspipantien) reduzieren durch Beeinflussung der Aktivität der ekkrinen Schweißdrüsen die Schweißbildung und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe
- Ölkomponenten
- nichtionische Emulgatoren
- Coemulgatoren
- Konsistenzgeber
- Hilfsstoffe wie z. B. Verdicker oder Komplexierungsmittel und/oder
- nichtässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat,Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringen Mengen enthalten sein. Solche öllöslichen Hilfsmittel können z. B. sein:
- entzündungshemmende, hautschützende oder wohlriechende ätherische Öle
- synthetische hautschützende Wirkstoffe und/oder
- öllösliche Parfumöle

Übliche wasserlösliche Zusätze sind z. B. wasserlösliche Duftstoffe, pH-Wert-Stellmittel, z. B. Puffergemische, wasserlösliche Verdickungsmittel, z. B. wasserlösliche natürliche oder synthetische Polymere wie z. B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, VinylpyrrolidonVinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimethylpentyl)-2-(1H)-pyridinonmonoethanolaminsalz), Baypival® (Climbazole), Ketoconazol®, (4-Acetyl-1-{-4-[2-(2.4-dichlorphenyl)-r-2-(1H-imidazol-1-ylmethyl)-1,3-dioxylan-c-4-ylmethoxyphenyl}piperazin, Elubiol, Selendisulfid, Schwefel kolloidal, Schwefelpolyethylenglykolsorbitanmonooleat, Schwefelricinolpolyethoxylat, Schwefel-Teer Destillate, Salicylsäure (bzw. in Kombination mit Hexachlorophen), Undexylensäure Monoethanolamid Sulfosuccinat Na-Salz, Lampeon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wässrige Phasen können Montmorillonite, Clay Mineralstoffe, Pemulen sowie alkyl-modifizierte Carbopoltypen (Goodrich) dienen. Weitere geeignete Polymere bzw. Quellmittel können der Übersicht von R. Lochhead in Cosm. Toil. 108, 95 (1993) entnommen werden.

### Insekt-Repellentien

Als Insekt-Repellentien kommen beispielsweise N,N-Diethyl-m-toluamid, 1,2-Pentandiol oder 3-(N-n-Butyl-N-acetyl-amino)-propionic aid ethyl ester), welches unter der Bezeichnung Insect Repellent® 3535 von der Merck KGaA vertrieben wird, sowie Butylacetylaminopropionate in Frage.

### Selbstbräuner und Depigmentierungsmittel

Als Selbstbräuner eignet sich Dihydroxyaceton. Als Tyrosininhibitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Typische Beispiele sind:
- Glycerin
- Alkylenglycole, wie beispielsweise Ethylenglycol, Diethylenglycol, Propylenglycol, Butylenglycol, Hexylenglycol, 1,2-Hexandiol und Caprylyl Glycol sowie Polyethylenglycole mit einem durchschnittlichen Molekulargewicht von 100 bis 1.000 Dalton.
- technische Oligoglyceringemische mit einem Eigenkondensationsgrad von 1,5 bis 10 wie etwa technische Diglyceringemische mit einem Diglyceringehalt von 40 bis 50 Gew.-%
- Methylolverbindungen, wie insbesondere Trimethylolethan, Trimethylolpropan, Trimethylolbutan, Pentaerythrit und Dipentarythrit
- Niedriglkylglucoside, insbesondere solche mit 1 bis 8 Kohlenstoffen im Alkylrest, wie bespielsweise Methyl- und Butylglucosid
- Zuckeralkohole mit 5 bis 12 Kohlenstoffatomen, wie beispiele Sorbit oder Mannit
- Zucker mit 5 bis 12 Kohlenstoffatomen, wie beispielsweise Glucose oder Saccharose
- Aminozucker, wie beispielsweise Glucamin
- Dialkoholamine, wie Diethanolamin oder 2-Amino-1,3-propandiol

### Parfümöle und Aromen

Als Parfümöle ein genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange),Wurzeln (Macis, Angelica, Sellerie, Kardamom, Costus, Iris, Calmus), Hölzern (Pinien-Sandel- Guajak-, Zedern-, Rosenholz), Kräutern und Grsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jojone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambraxon, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt.

Aus der Gruppe der organischen Säuren seien beispielhaft genannt Lävulinsäure, Zimtsäure oder Anissäure.

Als Aromen kommen beispielsweise Pfefferminzöl, Krauseminzöl, Anisöl, Sternanisöl, Kümmelöl, Eukalyptusöl, Fenchelöl, Zitronenöl, Wintergrünöl, Nelkenöl, Menthol und dergleichen in Frage.

Es sei ausdrücklich darauf hingewiesen, dass einige Aromen und Duftstoffe selbst antimikrobielle Eigenschaften aufweisen oder in Kombination mit Konservierungsmitteln oder Konservierungsmittelhelfern deren antimikrobielle Wirkung synergistisch verstärken können. Die synergistischen Wirkungen dieser Duftstoffe sind ausdrücklich in dieser Patentschrift inbegriffen.

### Farbstoffe

Als Farbstoffe können die für kosmetische Zwecke geeigneten und zugelassenen Substanzen verwendet werden. Beispiele sind Kochenillerot A (C.I. 16255), Patentblau V (C.I. 42051), Indigotin (C.I. 73015), Chlorophyllin (C.I. 75810), Chinolongelb (C.I. 47005), Titandioxid (C.I. 77891), Indanthrenblau RS (C.I. 69800) und Krapplack (C.I. 58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Die erfindungsgemäßen Formulierungen können dem Stand der Technik entsprechend hergestellt werden. Bevorzugt im Sinne der Erfindung sind kosmetische und/oder pharmazeutische Zubereitungen, die
(a) 0,01 - 50 Gew.-% Konservierungsmittelsystem aus zyklischen Carbonaten und Konservierungsmitteln
(b) 0,1 - 20 Gew.-% Tenside und/oder Emulgatoren und/oder Coemulgatoren
(c) 0,1 - 90 Gew.-% Ölkörper
(d) 0 - 98 Gew.-% Wasser
bezogen auf die Gesamtzusammensetzung enthalten.

Nachfolgend werden einige Beispiele genannt, die die vorliegende Erfindung verdeutlichen sollen, ohne diese einzuschränken.
1. Beispielrezepturen von Kombinationen zyklischer 1,2-Alkylencarbonat mit Dragocid Liquid (Phenoxyethanol, Methylparaben, Ethylparaben, Propylparaben, Butylparaben, Isobutylparaben).

| Phase | Handelsname | Dragocid Liquid | Dragocid Liquid / BC | Dragocid Liquid / PC | Dragocid Liquid / HC | Dragocid Liquid / OC |
|---|---|---|---|---|---|---|
| A | Sunflower Oil | 6,00 | 6,00 | 6,00 | 6,00 | 6,00 |
| | Myritol 318 | 6,00 | 6,00 | 6,00 | 6,00 | 6,00 |
| | Cetiol V | 6,00 | 6,00 | 6,00 | 6,00 | 6,00 |
| | Dermofeel® PS | 2,00 | 2,00 | 2,00 | 2,00 | 2,00 |
| | Dermofeel® SL | 2,00 | 2,00 | 2,00 | 2,00 | 2,00 |
| | Heliogel | 2,00 | 2,00 | 2,00 | 2,00 | 2,00 |
| | Dermofeel Toco 70 | 0,10 | 0,10 | 0,10 | 0,10 | 0,10 |
| | Dragocid Liquid | 0,50 | 0,50 | 0,50 | 0,50 | 0,50 |
| | 1,2-Butylencarbonat (BC) | - | 2,00 | - | - | - |
| | 1,2-Pentylencarbonat (PC) | - | - | 2,00 | - | - |
| | 1,2-Hexylencarbonat (HC) | - | - | - | 2,00 | - |
| | 1,2-Octylencarbonat (OC) | - | - | - | - | 2,00 |
| B | Demineralized Wasser | 71,30 | 69,30 | 69,30 | 69,30 | 69,30 |
| | Glycerin | 2,00 | 2,00 | 2,00 | 2,00 | 2,00 |
| | Dermofeel® PA 3 | 0,10 | 0,10 | 0,10 | 0,10 | 0,10 |
| | | **100,00** | **100,00** | **100,00** | **100,00** | **100,00** |

| | | | | | | |
|---|---|---|---|---|---|---|
| Myritol 318: Caprylic/Capric Triglycerides, Cetiol V: Decyl Oleate, Dermofeel PS: Polyglyceryl-3-Stearate, Dermofeel SL: Sodium Stearoyl Lactylate, Heliogel: Sodium Acrylates Copolymer, Hydrogenated Polyisobutene; Phospholipids; Polyglyceryl-10 Stearate; Helianthus Annuus, Dermofeel Toco 70: Tocopherol, Dermofeel PA-3: Sodium Phytate. | | | | | | |

Ergebnisse des mikrobiologischen Belastungstests, durchgeführt nach europäischem Arzneibuch 1997: 5.1.3 Prüfung auf ausreichende Konservierung (entspricht Deutschem Arzneibuch: VIII.14) für die Beispielrezeptur nach 1.:

| Koloniebildende Einheiten / g nach Beimpfung mit: | | | | | |
|---|---|---|---|---|---|
| Staphylococcus aureus | Dragocid Liquid | Dragocid Liquid / BC | Dragocid Liquid / PC | Dragocid Liquid / HC | Dragocid Liquid / OC |
| 0 Tage | 460000 | 460000 | 460000 | 460000 | 460000 |
| 2 Tage | 70000 | 7400 | 500 | 11000 | 120000 |
| 7 Tage | 3000 | <10 | <10 | <10 | 2000 |
| 14 Tage | <10 | <10 | <10 | <10 | <10 |
| 28 Tage | <10 | <10 | <10 | <10 | <10 |
| | | | | | |

| Pseudomonas Aeruginosa | | | | | |
|---|---|---|---|---|---|
| 0 Tage | 1800000 | 1800000 | 1800000 | 1800000 | 1800000 |
| 2 Tage | 1600 | <10 | <10 | <10 | <10 |
| 7 Tage | 200 | <10 | <10 | <10 | <10 |
| 14 Tage | 40 | <10 | <10 | <10 | <10 |
| 28 Tage | <10 | <10 | <10 | <10 | <10 |
| | | | | | |

| Escherichia Coli | | | | | |
|---|---|---|---|---|---|
| 0 Tage | 3400000 | 3400000 | 3400000 | 3400000 | 3400000 |
| 2 Tage | 100000 | 100000 | <10 | 10000 | 120000 |
| 7 Tage | 120000 | 11000 | <10 | <10 | 4000 |
| 14 Tage | 55000 | <10 | <10 | <10 | 120 |
| 28 Tage | 15000 | <10 | <10 | <10 | <10 |
| | | | | | |

| Candida Albicans | | | | | |
|---|---|---|---|---|---|
| 0 Tage | 620000 | 620000 | 620000 | 620000 | 620000 |
| 2 Tage | 80000 | 4400 | 2200 | 2400 | 13000 |
| 7 Tage | 80000 | <10 | <10 | <10 | 270 |
| 14 Tage | 80000 | <10 | <10 | <10 | <10 |
| 28 Tage | 82000 | <10 | <10 | <10 | <10 |
| | | | | | |

| Aspergillus Niger | | | | | |
|---|---|---|---|---|---|
| 0 Tage | 410000 | 410000 | 410000 | 410000 | 410000 |
| 2 Tage | 62000 | 15000 | 29000 | 42000 | 26000 |
| 7 Tage | 60000 | 10000 | 10000 | 40000 | 25000 |
| 14 Tage | 55000 | 100 | 800 | 12000 | 27000 |
| 28 Tage | 35000 | <10 | <10 | 4000 | 25000 |

2. Beispielrezepturen von Kombinationen zyklischer 1,2-Alkylencarbonat mit Optiphen (Phenoxyethanol, Caprylyl Glycol).

| Phase | Handelsname | Optiphen | Optiphen / BC | Optiphen / PC | Optiphen / HC | Optiphen / OC |
|---|---|---|---|---|---|---|
| A | Sunflower Oil | 6,00 | 6,00 | 6,00 | 6,00 | 6,00 |
| | Myritol 318 | 6,00 | 6,00 | 6,00 | 6,00 | 6,00 |
| | Cetiol V | 6,00 | 6,00 | 6,00 | 6,00 | 6,00 |
| | Dermofeel® PS | 2,00 | 2,00 | 2,00 | 2,00 | 2,00 |
| | Dermofeel® SL | 2,00 | 2,00 | 2,00 | 2,00 | 2,00 |
| | Heliogel | 2,00 | 2,00 | 2,00 | 2,00 | 2,00 |
| | Dermofeel Toco 70 | 0,10 | 0,10 | 0,10 | 0,10 | 0,10 |
| | Optiphen | 0,50 | 0,50 | 0,50 | 0,50 | 0,50 |
| | 1,2-Butylencarbonat (BC) | - | 2,00 | - | - | - |
| | 1,2-Pentylencarbonat (PC) | - | - | 2,00 | - | - |
| | 1,2-Hexylencarbonat (HC) | - | - | - | 2,00 | - |
| | 1,2-Octylencarbonat (OC) | - | - | - | - | 2,00 |
| B | Demineralized Wasser | 71,30 | 69,30 | 69,30 | 69,30 | 69,30 |
| | Glycerin | 2,00 | 2,00 | 2,00 | 2,00 | 2,00 |
| | Dermofeel® PA 3 | 0,10 | 0,10 | 0,10 | 0,10 | 0,10 |
| | | **100,00** | **100,00** | **100,00** | **100,00** | **100,00** |

| | | | | | | |
|---|---|---|---|---|---|---|
| Myritol 318: Caprylic/Capric Triglycerides, Cetiol V: Decyl Oleate, Dermofeel PS: Polyglyceryl-3-Stearate, Dermofeel SL: Sodium Stearoyl Lactylate, Heliogel: Sodium Acrylates Copolymer, Hydrogenated Polyisobutene; Phospholipids; Polyglyceryl-10 Stearate; Helianthus Annuus, Dermofeel Toco 70: Tocopherol, Dermofeel PA-3: Sodium Phytate. Ergebnisse des mikrobiologischen Belastungstests, durchgeführt nach europäischem Arzneibuch 1997: 5.1.3 Prüfung auf ausreichende Konservierung (entspricht Deutschem Arzneibuch: VIII.14) für die Beispielrezeptur nach 2.: | | | | | | |

| Koloniebildende Einheiten / g nach Beimpfung mit: | | | | | |
|---|---|---|---|---|---|
| Staphylococcus aureus | Optiphen | Optiphen / BC | Optiphen / PC | Optiphen / HC | Optiphen / OC |
| 0 Tage | 460000 | 460000 | 460000 | 460000 | 460000 |
| 2 Tage | 120000 | 32000 | 2400 | 8200 | 120000 |
| 7 Tage | 60000 | <10 | <10 | <10 | 17000 |
| 14 Tage | 500 | <10 | <10 | <10 | <10 |
| 28 Tage | <10 | <10 | <10 | <10 | <10 |
| | | | | | |

| Pseudomonas Aeruginosa | | | | | |
|---|---|---|---|---|---|
| 0 Tage | 1800000 | 1800000 | 1800000 | 1800000 | 1800000 |
| 2 Tage | <10 | <10 | <10 | <10 | 10 |
| 7 Tage | <10 | <10 | <10 | <10 | <10 |
| 14 Tage | <10 | <10 | <10 | <10 | <10 |
| 28 Tage | <10 | <10 | <10 | <10 | <10 |
| | | | | | |

| Escherichia Coli | | | | | |
|---|---|---|---|---|---|
| 0 Tage | 3400000 | 3400000 | 3400000 | 3400000 | 3400000 |
| 2 Tage | 12000 | 2800 | <10 | <10 | 40000 |
| 7 Tage | 15000 | <10 | <10 | <10 | <10 |
| 14 Tage | 340 | <10 | <10 | <10 | <10 |
| 28 Tage | <10 | <10 | <10 | <10 | <10 |
| | | | | | |

| Candida Albicans | | | | | |
|---|---|---|---|---|---|
| 0 Tage | 620000 | 620000 | 620000 | 620000 | 620000 |
| 2 Tage | 80000 | 4100 | 2400 | 900 | 6200 |
| 7 Tage | 50000 | <10 | <10 | <10 | <10 |
| 14 Tage | 50000 | <10 | <10 | <10 | <10 |
| 28 Tage | 45000 | <10 | <10 | <10 | <10 |
| | | | | | |

| Aspergillus Niger | | | | | |
|---|---|---|---|---|---|
| 0 Tage | 410000 | 410000 | 410000 | 410000 | 410000 |
| 2 Tage | 36000 | 11000 | 26000 | 50000 | 54000 |
| 7 Tage | 35000 | 12000 | 20000 | 30000 | 50000 |
| 14 Tage | 35000 | 8000 | 8000 | 30000 | 50000 |
| 28 Tage | 37000 | 140 | 650 | 32000 | 46000 |

3. Beispielrezepturen von Kombinationen zyklischer 1,2-Alkylencarbonat mit Phenoxyethanol

| Phase | Handelsname | Phenoxy ethanol | Phenoxy ethanol / BC | Phenoxy ethanol / PC | Phenoxy ethanol / HC | Phenoxy ethanol / OC |
|---|---|---|---|---|---|---|
| A | Sunflower Oil | 6,00 | 6,00 | 6,00 | 6,00 | 6,00 |
| | Myritol 318 | 6,00 | 6,00 | 6,00 | 6,00 | 6,00 |
| | Cetiol V | 6,00 | 6,00 | 6,00 | 6,00 | 6,00 |
| | Dermofeel® PS | 2,00 | 2,00 | 2,00 | 2,00 | 2,00 |
| | Dermofeel® SL | 2,00 | 2,00 | 2,00 | 2,00 | 2,00 |
| | Heliogel | 2,00 | 2,00 | 2,00 | 2,00 | 2,00 |
| | Dermofeel Toco 70 | 0,10 | 0,10 | 0,10 | 0,10 | 0,10 |
| | Phenoxyethanol | 0,50 | 0,50 | 0,50 | 0,50 | 0,50 |
| | 1,2-Butylencarbonat (BC) | - | 2,00 | - | - | - |
| | 1,2-Pentylencarbonat (PC) | - | - | 2,00 | - | - |
| | 1,2-Hexylencarbonat (HC) | - | - | - | 2,00 | - |
| | 1,2-Octylencarbonat (OC) | - | - | - | - | 2,00 |
| B | Demineralized Wasser | 71,30 | 69,30 | 69,30 | 69,30 | 69,30 |
| | Glycerin | 2,00 | 2,00 | 2,00 | 2,00 | 2,00 |
| | Dermofeel® PA 3 | 0,10 | 0,10 | 0,10 | 0,10 | 0,10 |
| | | **100,00** | **100,00** | **100,00** | **100,00** | **100,00** |

| | | | | | | |
|---|---|---|---|---|---|---|
| Myritol 318: Caprylic/Capric Triglycerides, Cetiol V: Decyl Oleate, Dermofeel PS: Polyglyceryl-3-Stearate, Dermofeel SL: Sodium Stearoyl Lactylate, Heliogel: Sodium Acrylates Copolymer, Hydrogenated Polyisobutene; Phospholipids; Polyglyceryl-10 Stearate; Helianthus Annuus, Dermofeel Toco 70: Tocopherol, Dermofeel PA-3: Sodium Phytate. | | | | | | |

Ergebnisse des mikrobiologischen Belastungstests, durchgeführt nach europäischem Arzneibuch 1997: 5.1.3 Prüfung auf ausreichende Konservierung (entspricht Deutschem Arzneibuch: VIII.14) für die Beispielrezeptur nach 3.:

| Koloniebildende Einheiten / g nach Beimpfung mit: | | | | | |
|---|---|---|---|---|---|
| Staphylococcus aureus | Phenoxyethanol | Phenoxyethanol / BC | Phenoxyethanol / PC | Phenoxyethanol / HC | Phenoxyethanol / OC |
| 0 Tage | 380000 | 380000 | 380000 | 380000 | 380000 |
| 2 Tage | 100000 | 100000 | 100000 | 100000 | 100000 |
| 7 Tage | 100000 | 72000 | 11000 | 14000 | 100000 |
| 14 Tage | 84000 | 3000 | 120 | 240 | 3000 |
| 28 Tage | 80000 | <10 | <10 | <10 | <10 |
| | | | | | |

| Pseudomonas Aeruginosa | | | | | |
|---|---|---|---|---|---|
| 0 Tage | 600000 | 600000 | 600000 | 600000 | 600000 |
| 2 Tage | 8000 | <10 | 3000 | <10 | 3000 |
| 7 Tage | 10000 | <10 | <10 | <10 | 10000 |
| 14 Tage | 10000 | <10 | <10 | <10 | 30000 |
| 28 Tage | 10000 | <10 | <10 | <10 | 40000 |
| | | | | | |

| Escherichia Coli | | | | | |
|---|---|---|---|---|---|
| 0 Tage | 3000000 | 300000 | 3000000 | 3000000 | 3000000 |
| 2 Tage | 100000 | 100000 | 100000 | 100000 | 100000 |
| 7 Tage | 100000 | 18000 | <10 | <10 | 100000 |
| 14 Tage | 100000 | <10 | <10 | <10 | 3000 |
| 28 Tage | 70000 | <10 | <10 | <10 | <10 |
| | | | | | |

| Candida Albicans | | | | | |
|---|---|---|---|---|---|
| 0 Tage | 310000 | 310000 | 310000 | 310000 | 310000 |
| 2 Tage | 100000 | 50000 | 20000 | 10000 | 40000 |
| 7 Tage | 100000 | <10 | <10 | <10 | 12000 |
| 14 Tage | 100000 | <10 | <10 | <10 | 1000 |
| 28 Tage | 80000 | <10 | <10 | <10 | <10 |
| | | | | | |

| Aspergillus Niger | | | | | |
|---|---|---|---|---|---|
| 0 Tage | 320000 | 320000 | 320000 | 320000 | 320000 |
| 2 Tage | 60000 | 50000 | 50000 | 60000 | 20000 |
| 7 Tage | 60000 | 30000 | 40000 | 60000 | 20000 |
| 14 Tage | 60000 | 1000 | 3000 | 40000 | 30000 |
| 28 Tage | 50000 | <10 | 160 | 32000 | 28000 |

4. Beispielrezepturen von Kombinationen zyklischer 1,2-Alkylencarbonat mit Rokonsal BS (Benzoic acid, Sorbic acid)

| Phase | Handelsname | Rokonsal BS | Rokonsal BS / BC | Rokonsal BS / PC | Rokonsal BS / HC | Rokonsal BS / OC |
|---|---|---|---|---|---|---|
| A | Sunflower Oil | 6,00 | 6,00 | 6,00 | 6,00 | 6,00 |
| | Myritol 318 | 6,00 | 6,00 | 6,00 | 6,00 | 6,00 |
| | Cetiol V | 6,00 | 6,00 | 6,00 | 6,00 | 6,00 |
| | Dermofeel® PS | 2,00 | 2,00 | 2,00 | 2,00 | 2,00 |
| | Dermofeel® SL | 2,00 | 2,00 | 2,00 | 2,00 | 2,00 |
| | Heliogel | 2,00 | 2,00 | 2,00 | 2,00 | 2,00 |
| | Dermofeel Toco 70 | 0,10 | 0,10 | 0,10 | 0,10 | 0,10 |
| | Rokonsal BS | 0,50 | 0,50 | 0,50 | 0,50 | 0,50 |
| | 1,2-Butylencarbonat (BC) | - | 2,00 | - | - | - |
| | 1,2-Pentylencarbonat (PC) | - | - | 2,00 | - | - |
| | 1,2-Hexylencarbonat (HC) | - | - | - | 2,00 | - |
| | 1,2-Octylencarbonat (OC) | - | - | - | - | 2,00 |
| B | Demineralized Wasser | 71,30 | 69,30 | 69,30 | 69,30 | 69,30 |
| | Glycerin | 2,00 | 2,00 | 2,00 | 2,00 | 2,00 |
| | Dermofeel® PA 3 | 0,10 | 0,10 | 0,10 | 0,10 | 0,10 |
| | | **100,00** | **100,00** | **100,00** | **100,00** | **100,00** |

| | | | | | | |
|---|---|---|---|---|---|---|
| Myritol 318: Caprylic/Capric Triglycerides, Cetiol V: Decyl Oleate, Dermofeel PS: Polyglyceryl-3-Stearate, Dermofeel SL: Sodium Stearoyl Lactylate, Heliogel: Sodium Acrylates Copolymer, Hydrogenated Polyisobutene; Phospholipids; Polyglyceryl-10 Stearate; Helianthus Annuus, Dermofeel Toco 70: Tocopherol, Dermofeel PA-3: Sodium Phytate. | | | | | | |

Ergebnisse des mikrobiologischen Belastungstests, durchgeführt nach europäischem Arzneibuch 1997: 5.1.3 Prüfung auf ausreichende Konservierung (entspricht Deutschem Arzneibuch: VIII.14) für die Beispielrezeptur nach 4.:

| Koloniebildende Einheiten / g nach Beimpfung mit: | | | | | |
|---|---|---|---|---|---|
| Staphylococcus aureus | Rokonsal BS | Rokonsal BS / BC | Rokonsal BS / PC | Rokonsal BS / HC | Rokonsal BS / OC |
| 0 Tage | 250000 | 250000 | 250000 | 250000 | 250000 |
| 2 Tage | 100000 | 50000 | 10000 | 15000 | 85000 |
| 7 Tage | 100000 | 11000 | 3000 | 8000 | 70000 |
| 14 Tage | 50000 | 300 | <10 | <10 | 30000 |
| 28 Tage | 25000 | <10 | <10 | <10 | 15000 |
| | | | | | |

| Pseudomonas Aeruginosa | | | | | |
|---|---|---|---|---|---|
| 0 Tage | 210000 | 210000 | 210000 | 210000 | 210000 |
| 2 Tage | 40000 | <10 | <10 | <10 | 3000 |
| 7 Tage | 5000 | <10 | <10 | <10 | <10 |
| 14 Tage | 300 | <10 | <10 | <10 | <10 |
| 28 Tage | <10 | <10 | <10 | <10 | <10 |
| | | | | | |

| Escherichia Coli | | | | | |
|---|---|---|---|---|---|
| 0 Tage | 810000 | 810000 | 810000 | 810000 | 810000 |
| 2 Tage | 100000 | 100000 | 80000 | 10000 | 100000 |
| 7 Tage | 100000 | 5000 | 3000 | 1500 | 60000 |
| 14 Tage | 65000 | <10 | <10 | <10 | 4000 |
| 28 Tage | 40000 | <10 | <10 | <10 | <10 |
| | | | | | |

| Candida Albicans | | | | | |
|---|---|---|---|---|---|
| 0 Tage | 410000 | 410000 | 410000 | 410000 | 410000 |
| 2 Tage | 100000 | 80000 | 100000 | 90000 | 100000 |
| 7 Tage | 100000 | 5000 | <10 | <10 | 12000 |
| 14 Tage | 100000 | <10 | <10 | <10 | 1000 |
| 28 Tage | 80000 | <10 | <10 | <10 | <10 |
| | | | | | |

| Aspergillus Niger | | | | | |
|---|---|---|---|---|---|
| 0 Tage | 170000 | 170000 | 170000 | 170000 | 170000 |
| 2 Tage | 60000 | 60000 | 30000 | 55000 | 60000 |
| 7 Tage | 60000 | 30000 | 12000 | 52000 | 58000 |
| 14 Tage | 60000 | 4000 | 6000 | 16000 | 58000 |
| 28 Tage | 49000 | <10 | 360 | 10000 | 56000 |

5. Beispielrezepturen von Kombinationen zyklischer 1,2-Alkylencarbonat mit Benzylalkohol

| Phase | Handelsname | Benzylalkohol | Benzylalkohol / BC | Benzylalkohol / PC | Benzylalkohol / HC | Benzylalkohol / OC |
|---|---|---|---|---|---|---|
| A | Sunflower Oil | 6,00 | 6,00 | 6,00 | 6,00 | 6,00 |
| | Myritol 318 | 6,00 | 6,00 | 6,00 | 6,00 | 6,00 |
| | Cetiol V | 6,00 | 6,00 | 6,00 | 6,00 | 6,00 |
| | Dermofeel® PS | 2,00 | 2,00 | 2,00 | 2,00 | 2,00 |
| | Dermofeel® SL | 2,00 | 2,00 | 2,00 | 2,00 | 2,00 |
| | Heliogel | 2,00 | 2,00 | 2,00 | 2,00 | 2,00 |
| | Dermofeel Toco 70 | 0,10 | 0,10 | 0,10 | 0,10 | 0,10 |
| | Benzylalkohol | 0,50 | 0,50 | 0,50 | 0,50 | 0,50 |
| | 1,2-Butylencarbonat (BC) | - | 2,00 | - | - | - |
| | 1,2-Pentylencarbonat (PC) | - | - | 2,00 | - | - |
| | 1,2-Hexylencarbonat (HC) | - | - | - | 2,00 | - |
| | 1,2-Octylencarbonat (OC) | - | - | - | - | 2,00 |
| B | Demineralized Wasser | 71,30 | 69,30 | 69,30 | 69,30 | 69,30 |
| | Glycerin | 2,00 | 2,00 | 2,00 | 2,00 | 2,00 |
| | Dermofeel® PA 3 | 0,10 | 0,10 | 0,10 | 0,10 | 0,10 |
| | | **100,00** | **100,00** | **100,00** | **100,00** | **100,00** |

| | | | | | | |
|---|---|---|---|---|---|---|
| Myritol 318: Caprylic/Capric Triglycerides, Cetiol V: Decyl Oleate, Dermofeel PS: Polyglyceryl-3-Stearate, Dermofeel SL: Sodium Stearoyl Lactylate, Heliogel: Sodium Acrylates Copolymer, Hydrogenated Polyisobutene; Phospholipids; Polyglyceryl-10 Stearate; Helianthus Annuus, Dermofeel Toco 70: Tocopherol, Dermofeel PA-3: Sodium Phytate. | | | | | | |

Ergebnisse des mikrobiologischen Belastungstests, durchgeführt nach europäischem Arzneibuch 1997: 5.1.3 Prüfung auf ausreichende Konservierung (entspricht Deutschem Arzneibuch: VIII.14) für die Beispielrezeptur nach 5.:

| Koloniebildende Einheiten / g nach Beimpfung mit: | | | | | |
|---|---|---|---|---|---|
| Staphylococcus aureus | Benzylalkohol | Benzylalkohol / BC | Benzylalkohol / PC | Benzylalkohol / HC | Benzylalkohol / OC |
| 0 Tage | 250000 | 250000 | 250000 | 250000 | 250000 |
| 2 Tage | 100000 | 80000 | 50000 | 50000 | 100000 |
| 7 Tage | 4000 | 700 | <10 | <10 | 6000 |
| 14 Tage | <10 | <10 | <10 | <10 | <10 |
| 28 Tage | <10 | <10 | <10 | <10 | <10 |
| | | | | | |

| Pseudomonas Aeruginosa | | | | | |
|---|---|---|---|---|---|
| 0 Tage | 210000 | 210000 | 210000 | 210000 | 210000 |
| 2 Tage | 40000 | <10 | <10 | <10 | 3000 |
| 7 Tage | 12000 | <10 | <10 | <10 | <10 |
| 14 Tage | 1000 | <10 | <10 | <10 | <10 |
| 28 Tage | <10 | <10 | <10 | <10 | <10 |
| | | | | | |

| Escherichia Coli | | | | | |
|---|---|---|---|---|---|
| 0 Tage | 810000 | 810000 | 810000 | 810000 | 810000 |
| 2 Tage | 100000 | 100000 | 80000 | 70000 | 100000 |
| 7 Tage | 100000 | 26000 | <10 | <10 | 60000 |
| 14 Tage | 52000 | 1200 | <10 | <10 | 12000 |
| 28 Tage | 8000 | <10 | <10 | <10 | 800 |
| | | | | | |

| Candida Albicans | | | | | |
|---|---|---|---|---|---|
| 0 Tage | 410000 | 410000 | 410000 | 410000 | 410000 |
| 2 Tage | 70000 | 6000 | 1500 | 800 | 18000 |
| 7 Tage | 50000 | <10 | <10 | <10 | 2400 |
| 14 Tage | 50000 | <10 | <10 | <10 | <10 |
| 28 Tage | 45000 | <10 | <10 | <10 | <10 |
| | | | | | |

| Aspergillus Niger | | | | | |
|---|---|---|---|---|---|
| 0 Tage | 170000 | 170000 | 170000 | 170000 | 170000 |
| 2 Tage | 64000 | 40000 | 52000 | 60000 | 42000 |
| 7 Tage | 60000 | 34000 | 46000 | 60000 | 40000 |
| 14 Tage | 59000 | 12000 | 40000 | 54000 | 40000 |
| 28 Tage | 61000 | 1900 | 11000 | 50000 | 40000 |

6. Beispielrezepturen von Kombinationen zyklischer 1,2-Alkylencarbonat und Dermosoft LP (Capyryl Glycol, Glyceryl Caprylate, Glycerin, Phenylpropanol, Aqua).

| Phase | Handelsname | Dermosoft LP | Dermosoft LP / BC | Dermosoft LP / PC | Dermosoft LP / HC | Dermosoft LP / OC |
|---|---|---|---|---|---|---|
| A | Sunflower Oil | 6,00 | 6,00 | 6,00 | 6,00 | 6,00 |
| | Myritol 318 | 6,00 | 6,00 | 6,00 | 6,00 | 6,00 |
| | Cetiol V | 6,00 | 6,00 | 6,00 | 6,00 | 6,00 |
| | Dermofeel® PS | 2,00 | 2,00 | 2,00 | 2,00 | 2,00 |
| | Dermofeel® SL | 2,00 | 2,00 | 2,00 | 2,00 | 2,00 |
| | Heliogel | 2,00 | 2,00 | 2,00 | 2,00 | 2,00 |
| | Dermofeel Toco 70 | 0,10 | 0,10 | 0,10 | 0,10 | 0,10 |
| | Dermosoft LP | 0,50 | 0,50 | 0,50 | 0,50 | 0,50 |
| | 1,2-Butylencarbonat (BC) | - | 2,00 | - | - | - |
| | 1,2-Pentylencarbonat (PC) | - | - | 2,00 | - | - |
| | 1,2-Hexylencarbonat (HC) | - | - | - | 2,00 | - |
| | 1,2-Octylencarbonat (OC) | - | - | - | - | 2,00 |
| B | Demineralized Wasser | 71,30 | 69,30 | 69,30 | 69,30 | 69,30 |
| | Glycerin | 2,00 | 2,00 | 2,00 | 2,00 | 2,00 |
| | Dermofeel® PA 3 | 0,10 | 0,10 | 0,10 | 0,10 | 0,10 |
| | | **100,00** | **100,00** | **100,00** | **100,00** | **100,00** |

| | | | | | | |
|---|---|---|---|---|---|---|
| Myritol 318: Caprylic/Capric Triglycerides, Cetiol V: Decyl Oleate, Dermofeel PS: Polyglyceryl-3-Stearate, Dermofeel SL: Sodium Stearoyl Lactylate, Heliogel: Sodium Acrylates Copolymer, Hydrogenated Polyisobutene; Phospholipids; Polyglyceryl-10 Stearate; Helianthus Annuus, Dermofeel Toco 70: Tocopherol, Dermofeel PA-3: Sodium Phytate. | | | | | | |

Ergebnisse des mikrobiologischen Belastungstests, durchgeführt nach europäischem Arzneibuch 1997: 5.1.3 Prüfung auf ausreichende Konservierung (entspricht Deutschem Arzneibuch: VIII.14) für die Beispielrezeptur nach 6.:

| Koloniebildende Einheiten / g nach Beimpfung mit: | | | | | |
|---|---|---|---|---|---|
| Staphylococcus aureus | Dermosoft LP | Dermosoft LP/ BC | Dermosoft LP/ PC | Dermosoft LP/ HC | Dermosoft LP/ OC |
| 0 Tage | 250000 | 250000 | 250000 | 250000 | 250000 |
| 2 Tage | 36000 | 11000 | 1500 | 9000 | 38000 |
| 7 Tage | 1000 | <10 | <10 | <10 | 600 |
| 14 Tage | <10 | <10 | <10 | <10 | <10 |
| 28 Tage | <10 | <10 | <10 | <10 | <10 |
| | | | | | |
| Pseudomonas Aeruginosa | | | | | |
| 0 Tage | 210000 | 210000 | 210000 | 210000 | 210000 |
| 2 Tage | <10 | <10 | <10 | <10 | <10 |
| 7 Tage | <10 | <10 | <10 | <10 | <10 |
| 14 Tage | <10 | <10 | <10 | <10 | <10 |
| 28 Tage | <10 | <10 | <10 | <10 | <10 |
| | | | | | |

| Escherichia Coli | | | | | |
|---|---|---|---|---|---|
| 0 Tage | 810000 | 810000 | 810000 | 810000 | 810000 |
| 2 Tage | 120000 | 40000 | <10 | 16000 | 40000 |
| 7 Tage | 35000 | 5600 | <10 | <10 | 10000 |
| 14 Tage | 2600 | <10 | <10 | <10 | <10 |
| 28 Tage | <10 | <10 | <10 | <10 | <10 |
| | | | | | |

| Candida Albicans | | | | | |
|---|---|---|---|---|---|
| 0 Tage | 410000 | 410000 | 410000 | 410000 | 410000 |
| 2 Tage | 60000 | 6100 | 400 | 1900 | 19000 |
| 7 Tage | 41000 | <10 | <10 | <10 | <10 |
| 14 Tage | 35000 | <10 | <10 | <10 | <10 |
| 28 Tage | 11000 | <10 | <10 | <10 | <10 |
| | | | | | |

| Aspergillus Niger | | | | | |
|---|---|---|---|---|---|
| 0 Tage | 170000 | 170000 | 170000 | 170000 | 170000 |
| 2 Tage | 25000 | 18000 | 20000 | 30000 | 26000 |
| 7 Tage | 15000 | 15000 | 16000 | 12000 | 25000 |
| 14 Tage | 15000 | 6000 | 7500 | 10000 | 27000 |
| 28 Tage | 10000 | <10 | 90 | 8000 | 15000 |

## Patentansprüche

1. Verwendung eines oder mehrerer zyklischer 1,2-Alkylencarbonate der allgemeinen Strukturformel 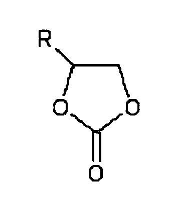 wobei R eine lineare oder verzweigte Alkylkette mit n = 2 - 8 Kohlenstoffatomen repräsentiert, zur Stabilisierung und Erhöhung der Wirksamkeit von einer oder mehreren das Wachstum von Mikroorganismen hemmenden Substanzen.

2. Verwendung nach Anspruch 1, **dadurch gekennzeichnet, dass** sie als Mischung in Form eines antimikrobiellen Stoffes zur biologischen Stabilisierung von kosmetischen oder dermatologischen Formulierungen zur Behandlung, Pflege oder Reinigung der Haut und / oder der Haare sowie in Produkten der dekorativen Kosmetik eingesetzt wird, **dadurch gekennzeichnet, dass** die Mischung in der fertigen kosmetischen oder dermatologischen Zubereitung in einer Konzentration von 0,01 - 50 Gewichts-%, bevorzugt 0,1 - 10,0 Gewichts-% bezogen auf das Gesamtgewicht der Formulierung enthalten ist.

3. Verwendung nach Anspruch 1 und/oder 2 als antimikrobieller Stoff, **dadurch gekennzeichnet, dass** die Mischung in Form einer kosmetischen Formulierung eine oder mehrere oberflächenaktive Substanzen und / oder eine oder mehrer Ölkörper enthält.

4. Verwendung nach Anspruch 1-3, **dadurch gekennzeichnet, dass** die Formulierung eine oder mehrere Duftstoffkomponenten aus der Gruppe der im EU Inventar gelisteten Duftstoffe enthält.

5. Mittel zur Stabilisierung und Erhöhung der Wirksamkeit von einer oder mehreren das Wachstum von Mikroorganismen hemmenden Substanzen (b), **gekennzeichnet durch** einen Zusatz von einem oder mehreren zyklischen 1,2-Alkylencarbonaten (a) der allgemein Strukturformel 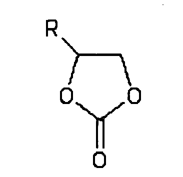 wobei R eine lineare oder verzweigte Alkylkette mit n = 2 - 8 Kohlenstoffatomen repräsentiert.

6. Mittel oder Mischung nach Anspruch 5, in denen b) besteht aus
- einer oder mehrerer Verbindungen aus der Gruppe der im Anhang VI der Kosmetikverordnung genannten Konservierungsmittel und/oder
- einer oder mehrerer multifunktioneller Kosmetikadditive mit antimikrobiellen Eigenschaften aus der Gruppe der Polyole (z.B. Pentylenglycol, 1.2-Hexandiol, Caprylyl Glycol, Methylpropandiol, Hexylenglycol), der Monoglyceride von Fettsäuren mit Kettenlängen von n = 6 - 12, (z.B. Glyceryl Caprylate, Glyceryl Caprate, Glyceryl Laurate), der linearen und verzweigte Alkylether des Glycerins (z.B. Ethylhexylglycerin) und / oder der N-acyl-Derivate von Aminosäuren (z.B. N-Undecylenoyl Glycin).

7. Mittel oder Mischung nach Anspruch 6, **dadurch gekennzeichnet, dass** die Verbindungen aus der Gruppe der im Anhang VI der Kosmetikverordnung genannten Konservierungsmittel bevorzugt gewählt werden aus der Gruppe: Benzoesäure, ihre Salze und Ester, Sorbinsäure und ihre Salze, p-Hydroxybenzoesäure, ihre Salze und Ester, 2-Phenoxyethanol und / oder Benzylalkohol und das die multifunktionellen Kosmetikadditive bevorzugt gewählt werden aus der Gruppe Caprylyl Glycol und Glyceryl Caprylate.

8. Mittel oder Mischung nach Anspruch 5 in denen das Verhältnis von a) zu b) im Bereich zwischen 100 : 1 bis 1 : 100 liegt.

9. Mittel oder Mischung nach Anspruch 8, **dadurch gekennzeichnet, dass** das Verhältnis von a) zu b) ein Bereich 10 : 1 bis 1 : 1 liegt.

10. Mittel oder Mischung nach Anspruch 5 in denen das zyklische 1,2-Alkylencarbonat aus der Gruppe 1,2-Butylencarbonat und / oder 1,2-Pentylencarbonat gewählt wird.

## Geänderte Patentansprüche

### Geänderte Patentansprüche gemäss Regel 86(2) EPÜ.

**1.** Mittel zur Stabilisierung und Erhöhung der Wirksamkeit von einer oder mehreren das Wachstum von Mikroorganismen hemmenden Substanzen (b), in denen (b) besteht aus
- einer oder mehrerer Verbindungen aus der Gruppe der im Anhang VI der Kosmetikverordnung genannten Konservierungsmittel und/oder
- einer oder mehrerer multifunktioneller Kosmetikadditive mit antimikrobiellen Eigenschaften aus der Gruppe der Polyole (z.B. Pentylenglycol, 1.2-Hexandiol, Caprylyl Glycol, Methylpropandiol, Hexylenglycol), der Monoglyceride von Fettsäuren mit Kettenlängen von n = 6 - 12, (z.B. Glyceryl Caprylate, Glyceryl Caprate, Glyceryl Laurate), der linearen und verzweigte Alkylether des Glycerins (z.B. Ethylhexylglycerin) und / oder der N-acyl-Derivate von Aminosäuren (z.B. N-Undecylenoyl Glycin) mit einem Zusatz von einem oder mehreren zyklischen 1,2-Alkylencarbonaten (a) der allgemein Strukturformel 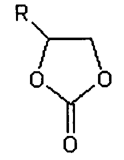 wobei R eine lineare oder verzweigte Alkylkette mit n = 2 - 8 Kohlenstoffatomen repräsentiert, **dadurch gekennzeichnet, dass** die Verbindungen aus der Gruppe der im Anhang VI der Kosmetikverordnung genannten Konservierungsmittel bevorzugt gewählt werden aus der Gruppe: Benzoesäure, ihre Salze und Ester, Sorbinsäure und ihre Salze, p-Hydroxybenzoesäure, ihre Salze und Ester, 2-Phenoxyethanol und / oder Benzylalkohol und das die multifunktionellen Kosmetikadditive bevorzugt gewählt werden aus der Gruppe Caprylyl Glycol und Glyceryl Caprylate.

**2.** Mittel oder Mischung nach Anspruch 1, in denen das Verhältnis von (a) zu (b) im Bereich zwischen 100 : 1 bis 1 : 100 liegt.

**3.** Mittel oder Mischung nach Anspruch 2, **dadurch gekennzeichnet, dass** das Verhältnis von (a) zu (b) im Bereich zwischen 10 : 1 bis 1 : 1 liegt.

**4.** Mittel oder Mischung nach Anspruch 1, in denen das zyklische 1,2-Alkylencarbonat aus der Gruppe 1,2-Butylencarbonat und/oder 1,2-Pentylencarbonat gewählt wird.

**5.** Verwendung des Mittels oder der Mischung nach einem oder mehreren der vorstehenden Ansprüche 1 - 4 zur biologischen Stabilisierung von kosmetischen oder dermatologischen Formulierungen zur Behandlung, Pflege oder Reinigung der Haut und / oder der Haare sowie in Produkten der dekorativen Kosmetik.
